# EUROPEAN PATENT SPECIFICATION

(11) **EP 1 942 945 B1**
(45) Date of publication and mention of the grant of the patent: **19.10.2011**
(21) Application number: 05851907.5
(22) Date of filing: 07.11.2005
(51) Int. Cl.: C12N 15/11, A61K 48/00

(54) **Immunostimulatory properties of oligonucleotide-based compounds comprising modified immunostimulatory dinucleotides**
Immunstimulatorische Eigenschaften von Verbindungen auf Oligonukleotid-basis mit modifizierten immunstimulatorischen Dinukleotiden
Propriétés immunostimulatrices de composés à base d'oligonucléotides comprenant des dinucléotides immunostimulateurs modifies

(43) Date of publication of application: 16.07.2008
(60) Divisional application: 11001257.2
(73) Proprietor: Idera Pharmaceuticals, Cambridge, MA 02139 (US)
(72) Inventor: AGRAWAL, Sudhir, Shrewsbury, MA 01545-5456 (US); YU, Dong, Westboro, MA 01581-1145 (US); KANDIMALLA, Ekambar, R., Southboro, MA 01772-1981 (US)
(74) Representative: Schnappauf, Georg
(86) International application number: PCT/US2005/042068
(87) International publication number: WO 2007/055704

(56) References cited:
- WO-A-03/057822
- WO-A-2004/064782
- US-A1- 2006 025 365
- KANDIMALLA E R ET AL: "TOWARDS OPTIMAL DESIGN OF SECOND-GENERATION IMMUNOMODULATORY OLIGONUCLEOTIDES" CURRENT OPINION IN MOLECULAR THERAPEUTICS, CURRENT DRUGS, LONDON, GB, vol. 4, no. 2, 1 January 2002 (2002-01-01), pages 122-129, XP009062237 ISSN: 1464-8431
- KANDIMALLA E R ET AL: "Divergent synthetic nucleotide motif recognition pattern: design and developmentof potent immunomodulatory oligodeoxyribonucleotide agents with distinct cytokine induction profiles" NUCLEIC ACIDS RESEARCH, OXFORD UNIVERSITY PRESS, SURREY, GB, vol. 31, no. 9, 1 January 2003 (2003-01-01), pages 2393-2400, XP002989074 ISSN: 0305-1048
- BHAGAT L ET AL: "CpG penta- and hexdeoxyribonucleotides as potent immunomodulatory agents" BIOCHEMICAL AND BIOPHYSICAL RESEARCH COMMUNICATIONS, ACADEMIC PRESS INC. ORLANDO, FL, US, vol. 300, 1 January 2003 (2003-01-01), pages 853-861, XP002989072 ISSN: 0006-291X
- LIANG ET AL.: 'Activation of Human B Cells by Phosphorotioate Oligodeoxynucleotides' J. CLIN. INVEST. vol. 98, no. 5, pages 1119 - 1129, XP002130608
- KANDIMALLA ET AL.: 'A dinucleotide motif in oligonucleotides shows potent immunomodulatory activity and overrides species-specific recognition observed with CpG motif' PNAS vol. 100, no. 24, 2003, pages 14303 - 14308, XP002990545
- BHAGAT ET AL.: 'CpG penta- and hexadeoxyribonucleotides as potent immunomodulatory agents' BIOCHEMICAL AND BIOPHYSICAL RESEARCH COMMUNICATIONS vol. 300, 2003, pages 853 - 861, XP002989072
- YU ET AL.: 'Immunomers-novel 3'-3'-linked CpG oligodeoxynucleotides as potent immunomodulatory agents' NUCLEIC ACIDS RESEARCH vol. 30, no. 20, 2002, pages 4460 - 4469, XP002903989

## Description

### Field of the Invention

The invention relates to immunology and immunotherapy applications using oligonucleotides as immunostimulatory agents.

### Summary of the Related Art

Oligonucleotides have become indispensable tools in modem molecular biology, being used in a wide variety of techniques, ranging from diagnostic probing methods to PCR to antisense inhibition of gene expression and immunotherapy applications. This widespread use of oligonucleotides has led to an increasing demand for rapid, inexpensive and efficient methods for synthesizing oligonucleotides.

The synthesis of oligonucleotides for antisense and diagnostic applications can now be routinely accomplished. See, e.g., Methods in Molecular Biology, Vol. 20: Protocols for Oligonucleotides and Analogs pp. 165-189 (S. Agrawal, ed., Humana Press, 1993); Oligonucleotides and Analogues, A Practical Approach, pp. 87-108 (F. Eckstein, ed., 1991); and Uhlmann and Peyman, *supra;* Agrawal and Iyer, Curr. Op. in Biotech. 6:12 (1995); and Antisense Research and Applications (Crooke and Lebleu, eds., CRC Press, Boca Raton, 1993). Early synthetic approaches included phosphodiester and phosphotriester chemistries. For example, Khorana et al., J. Molec. Biol. 72:209 (1972) discloses phosphodiester chemistry for oligonucleotide synthesis. Reese, Tetrahedron Lett. 34:3143-3179 (1978), discloses phosphotriester chemistry for synthesis of oligonucleotides and polynucleotides. These early approaches have largely given way to the more efficient phosphoramidite and H-phosphonate approaches to synthesis. For example, Beaucage and Caruthers, Tetrahedron Lett. 22:1859-1862 (1981), discloses the use of deoxyribonucleoside phosphoramidites in polynucleotide synthesis. Agrawal and Zamecnik, U.S. Patent No. 5,149,798 (1992), discloses optimized synthesis of oligonucleotides by the H-phosphonate approach. Both of these modern approaches have been used to synthesize oligonucleotides having a variety of modified internucleotide linkages. Agrawal and Goodchild, Tetrahedron Lett. 28:3539-3542 (1987), teaches synthesis of oligonucleotide methylphosphonates using phosphoramidite chemistry. Connolly et al., Biochem. 23:3443 (1984), discloses synthesis of oligonucleotide phosphorothioates using phosphoramidite chemistry. Jager et al., Biochem. 27:7237 (1988), discloses synthesis of oligonucleotide phosphoramidates using phosphoramidite chemistry. Agrawal et al., Proc. Natl. Acad Sci. (USA) 85:7079-7083 (1988), discloses synthesis of oligonucleotide phosphoramidates and phosphorothioates using H-phosphonate chemistry.

More recently, several researchers have demonstrated the validity of the use of oligonucleotides as immunostimulatory agents in immunotherapy applications. The observation that phosphodiester and phosphorothioate oligonucleotides can induce immune stimulation has created interest in developing this side effect as a therapeutic tool. These efforts have focused on phosphorothioate oligonucleotides containing the dinucleotide natural CpG. Kuramoto et al., Jpn. J. Cancer Res. 83:1128-1131 (1992) teaches that phosphodiester oligonucleotides containing a palindrome that includes a CpG dinucleotide can induce interferon alpha and gamma synthesis and enhance natural killer activity. Krieg et al., Nature 371:546-549 (1995) discloses that phosphorothioate CpG-containing oligonucleotides are immunostimulatory. Liang et al., J. Clin. Invest. 98:1119-1129 (1996) discloses that such oligonucleotides activate human B cells. Moldoveanu et al., Vaccine 16:1216-124 (1998) teaches that CpG-containing phosphorothioate oligonucleotides enhance immune response against influenza virus. McCluskie and Davis, J. Immunol. 161:4463-4466 (1998) teaches that CpG-containing oligonucleotides act as potent adjuvants, enhancing immune response against hepatitis B surface antigen. Hartman et al., J. Immunol 164:1617-1624 (2000) teaches that the immunostimulatory sequence is species specific, and different between mice and primates.

Other modifications of CpG-containing phosphorothioate oligonucleotides can also affect their ability to act as modulators of immune response. See, *e.g.,* Zhao et al., Biochem. Pharmacol. (1996) 51:173-182; Zhao et al., Biochem Phamiacol. (1996) 52:1537-1544; Zhao et al., Antisense Nucleic Acid Drug Dev. (1997) 7:495-502; Zhao et al., Bioorg. Med Chem. Lett. (1999) 9:3453-3458; Zhao et al., Bioorg. Med Chem. Lett. (2000) 10:1051-1054; Yu et al., Bioorg. Med Chem. Lett. (2000) 10:2585-2588; Yu et al., Bioorg. Med Chem. Lett. (2001) 11:2263-2267; and Kandimalla et al., Bioorg. Med Chem. (2001) 9:807-813.

These reports make clear that there remains a need to be able to modulate the immune response caused by immunostimulatory oligonucleotides and to overcome species specificity of the immunostimulatory sequences.

### BRIEF SUMMARY OF THE INVENTION

The invention provides means for modulating the immune response caused by oligonucleotide compounds. These means enable modifying the cytokine profile produced by immunostimulatory oligonucleotides for immunotherapy applications. The present inventors have surprisingly discovered that modification of immunostimulatory dinucleotides allows flexibility in the nature of the immune response produced and that certain modifications overcome the species specificities observed to date of the immunostimulatory sequences.

In a first aspect the invention provides an immunostimulatory oligonucleotide having a structure from the group of 5'-TCAGTCG₂TTAG-X-GATTG₂CTGACT-5' and 5'-TCAGTCG₁TTAG-X-GATTG₁CTGACT-5'; wherein G₁ = 2'-deoxy-7-deazaguanosine; G₂ = arabinoguanosine; and X = glycerol linker.

In a second aspect the invention provides a composition comprising the oligonucleotide disclosed in the first aspect of the invention.

In a third aspect the invention provides a pharmaceutical composition comprising the oligonucleotide disclosed in the first aspect of the invention and a physiologically acceptable carrier.

In a fourth aspect the invention provides an immunostimulatory oligonucleotide having a structure from the group of 5'-TCAGTCG₂TTAG-X-GATTG₂CTGACT-5' and 5'-TCAGTCG₁TTAG-X-GATTG₁CTGACT-5'; wherein wherein G₁ = 2'-deoxy-7-deazaguanosine; G₂ = arabinoguanosine; and X = glycerol linker for use in generating an immune response in a vertebrate.

In a fifth aspect the invention provides an immunostimulatory oligonucleotide having a structure from the group of 5'-TCAGTCG₂TTAG-X-GATTG₂CTGACT-5' and 5'-TCAGTCG₁TTAG-X-GATTG₁CTGACT-5'; wherein G₁ = 2'-deoxy-7-deazaguanosine; G₂ = arabinoguanosine; and X = glycerol linker for use in therapeutically treating a vertebrate having cancer, an autoimmune disorder, airway inflammation, inflammatory disorders, skin disorders, allergy, asthma or a disease caused by a pathogen.

In a sixth aspect the invention provides an immunostimulatory oligonucleotide having a structure from the group of 5'-TCAGTCG₂TTAG-X-GATTG₂CTGACT-5' and 5'-TCAGTCG₁TTAG-X-GATTG₁CTGACT-5'; wherein G₁ = 2'-deoxy-7-deazaguanosine; G₂ = arabinoguanosine; and X = glycerol linker for use in preventing cancer, an autoimmune disorder, airway inflammation, inflammatory disorders, skin disorders, allergy, asthma or a disease caused by a pathogen in a vertebrate.

In a seventh aspect the invention provides the use of an immunostimulatory oligonucleotide having a structure from the group of 5'-TCAGTCG₂TTAG-X-GATTG₂CTGACT-5' and 5'-TCAGTCG₁TTAG-X-GATTG₁CTGACT-5'; wherein G₁ = 2'-deoxy-7-deazaguanosine; G₂ = arabinoguanosine; and X = glycerol linker for the preparation of a pharmaceutical formulation for generating an immune response in a vertebrate.

In an eighth aspect the invention provides the use of an immunostimulatory oligonucleotide having a structure from the group of 5'-TCAGTCG₂TTAG-X-GATTG₂CTGACT-5' and 5'-TCAGTCG₁TTAG-X-GATTG₁CTGACT-5'; wherein G₁ = 2'-deoxy-7-deazaguanosine; G₂ = arabinoguanosine; and X = glycerol linker for the preparation of a pharmaceutical formulation for therapeutically treating a vertebrate having cancer, an autoimmune disorder, airway inflammation, inflammatory disorders, skin disorders, allergy, asthma or a disease caused by a pathogen.

In a ninth aspect the invention provides the use of an immunostimulatory oligonucleotide having a structure from the group of 5'-TCAGTCG₂TTAG-X-GATTG₂CTGACT-5' and 5'-TCAGTCG₁TTAG-X-GATTG₁CTGACT-5'; wherein G₁ = 2'-deoxy-7-deazaguanosine; G₂ = arabinoguanosine; and X = glycerol linker for the preparation of a pharmaceutical formulation for preventing cancer, an autoimmune disorder, airway inflammation, inflammatory disorders, skin disorders, allergy, asthma or a disease caused by a pathogen in a vertebrate.

Preferred embodiments of the aspects of the invention are set forth in claims 3, 5,7,9,11,12, and 16.

### BRIEF DESCRIPTION OF THE DRAWINGS

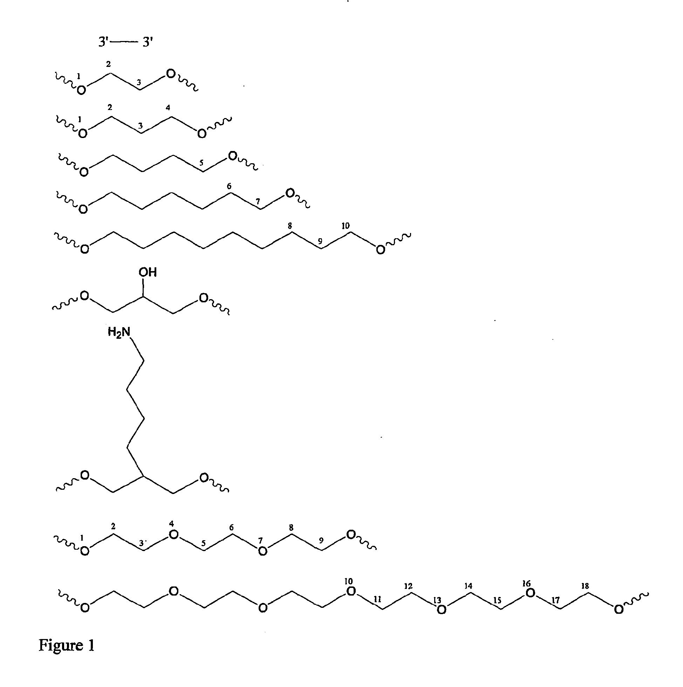

Figure 1 depicts a group of representative small molecule linkers suitable for linear synthesis of immunostimulatory oligonucleotides .

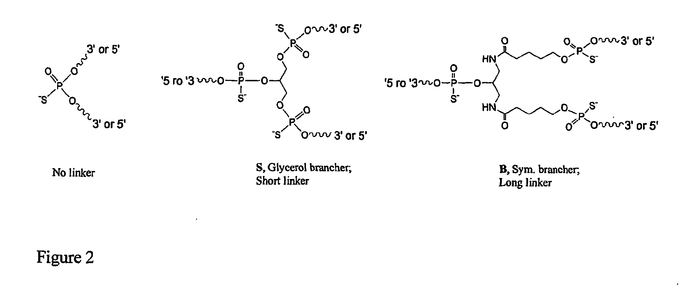

Figure 2 depicts a group of representative small molecule linkers suitable for parallel synthesis of immunostimulatory oligonucleotides .
Figure 3 is a synthetic scheme for the linear synthesis of immunostimulatory oligonucleotides of the invention. DMTr = 4,4'-dimethoxytrityl; CE = cyanoethyl.
Figure 4 is a synthetic scheme for the parallel synthesis of immunostimulatory oligonucleotides of the invention. DMTr = 4,4'-dimethoxytrityl; CE = cyanoethyl.
Figure 5 shows the activation of TLR9 by immunostimulatory oligonucleotides of the invention in HEK293 cells.
Figure 6 shows IFN-α induction in human pDC by immunostimulatory oligonucleotides of the invention.
Figure 7 shows IL-6 induction in human PBMCs by immunostimulatory oligonucleotides of the invention.
Figure 8 shows human B cell proliferation (72 hr) by immunostimulatory oligonucleotides of the invention.
Figure 9 shows IL-12 induction in C57BL/6 mouse spleen cell cultures by immunostimulatory oligonucleotides of the invention.
Figure 10 shows IL-6 induction in C57BL/6 mouse spleen cell cultures by immunostimulatory oligonucleotides of the invention.
Figure 11 is a schematic representation of the 3'-terminal nucleoside of an oligonucleotide, showing that a non-nucleotidic linkage can be attached to the nucleoside at the nucleobase, at the 3' position, or at the 2' position.

### DETAILED DESCRIPTION OF THE PREFERRED EMBODIMENTS

The invention relates to oligonucleotides which are therapeutically useful as immunostimulatory agents for immunotherapy applications. The issued patents, patent applications, and references that are cited herein are hereby incorporated by reference to the same extent as if each was specifically and individually indicated to be incorporated by reference. In the event of inconsistencies between any teaching of any reference cited herein and the present specification, the latter shall prevail for purposes of the invention.

The invention provides means for enhancing the immune response caused by immunostimulatory compounds used for immunotherapy applications such as, but not limited to, treatment of cancer, autoimmune disorders, asthma, respiratory allergies, food allergies, and bacteria, parasitic, and viral infections in adult and pediatric human and veterinary applications. Thus, the invention provides compounds having optimal levels of immunostimulatory effect for immunotherapy and methods for making such compounds. In addition, compounds of the invention are useful as adjuvants in combination with DNA vaccines, antibodies, and allergens; and in combination with chemotherapeutic agents and/or antisense oligonucleotides.

The present inventors have surprisingly discovered that, modification of an immunostimulatory oligonucleotide to optimally present its 5' ends dramatically affects its immunostimulatory capabilities. In addition, the present inventors have discovered that the cytokine profile and species specificity of an immune response can be modulated by using novel purine or pyrimidine structures as part of an immunostimulatory oligonucleotide.

As used herein, the term "accessible 5' end" means that the 5' end of the oligonucleotide is sufficiently available such that the factors that recognize and bind to oligonucleotide and stimulate the immune system have access to it. In oligonucleotides having an accessible 5' end, the 5' OH position of the terminal sugar is not covalently linked to more than two nucleoside residues or any other moiety that interferes with interaction with the 5' end. Optionally, the 5' OH can be linked to a phosphate, phosphorothioate, or phosphorodithioate moiety, an aromatic or aliphatic linker, cholesterol, or another entity which does not interfere with accessibility.

In certain embodiments, the immunostimulatory oligonucleotides include a ribozyme or a decoy oligonucleotide. As used herein, the term "ribozyme" refers to an oligonucleotide that possesses catalytic activity. Preferably, the ribozyme binds to a specific nucleic acid target and cleaves the target. As used herein, the term "decoy oligonucleotide" refers to an oligonucleotide that binds to a transcription factor in a sequence-specific manner and arrests transcription activity. Preferably, the ribozyme or decoy oligonucleotide exhibits secondary structure, including, without limitation, stem-loop or hairpin structures. In certain embodiments, at least one oligonucleotide comprises poly(I)-poly(C). In certain embodiments, at least one set ofNn includes a string of 3 to 10 dGs and/or Gs or 2'-substituted ribo or arabino Gs.

For purposes of the invention, the term "oligonucleotide" refers to a polynucleoside formed from a plurality of linked nucleoside units. Such oligonucleotides can be obtained from existing nucleic acid sources, including genomic or cDNA, but are preferably produced by synthetic methods. In preferred embodiments each nucleoside unit includes a heterocyclic base and a pentofuranosyl, trehalose, arabinose, 2'-deoxy-2'-substituted arabinose, 2'-O-substituted arabinose or hexose sugar group. The nucleoside residues can be coupled to each other by any of the numerous known internucleoside linkages. Such internucleoside linkages include, without limitation, phosphodiester, phosphorothioate, phosphorodithioate, alkylphosphonate, alkylphosphonothioate, phosphotriester, phosphoramidate, siloxane, carbonate, carboalkoxy, acetamidate, carbamate, morpholino, borano, thioether, bridged phosphoramidate, bridged methylene phosphonate, bridged phosphorothioate, and sulfone internucleoside linkages. The term "oligonucleotide" also encompasses polynucleosides having one or more stereospecific internucleoside linkage (e.g., (*R_{P}*)- or (*S_{P}*)-phosphorothioate, alkylphosphonate, or phosphotriester linkages). As used herein, the terms "oligonucleotide" and "dinucleotide" are expressly intended to include polynucleosides and dinucleosides having any such internucleoside linkage, whether or not the linkage comprises a phosphate group. In certain preferred embodiments, these internucleoside linkages may be phosphodiester, phosphorothioate, or phosphorodithioate linkages, or combinations thereof.

The term "oligonucleotide" also encompasses polynucleosides having additional substituents including, without limitation, protein groups, lipophilic groups, intercalating agents, diamines, folic acid, cholesterol and adamantane. The term "oligonucleotide" also encompasses any other nucleobase containing polymer, including, without limitation, peptide nucleic acids (PNA), peptide nucleic acids with phosphate groups (PHONA), locked nucleic acids (LNA), morpholino-backbone oligonucleotides, and oligonucleotides having backbone sections with alkyl linkers or amino linkers.

The oligonucleotides of the invention can include naturally occurring nucleosides, modified nucleosides, or mixtures thereof. As used herein, the term "modified nucleoside" is a nucleoside that includes a modified heterocyclic base, a modified sugar moiety, or a combination thereof In some embodiments, the modified nucleoside is a non-natural pyrimidine or purine nucleoside, as herein described. In some embodiments, the modified nucleoside is a 2'-substituted ribonucleoside, an arabinonucleoside or a 2'-deoxy-2'-substituted-arabinoside.

For purposes of the invention, the term "2'-substituted ribonucleoside" or "2'-substituted arabinoside" includes ribonucleosides or arabinonucleoside in which the hydroxyl group at the 2' position of the pentose moiety is substituted to produce a 2'-substituted or 2'-O-substituted ribonucleoside. Preferably, such substitution is with a lower alkyl group containing 1-6 saturated or unsaturated carbon atoms, or with an aryl group having 6-10 carbon atoms, wherein such alkyl, or aryl group may be unsubstituted or may be substituted, *e.g.*, with halo, hydroxy, trifluoromethyl, cyano, nitro, acyl, acyloxy, alkoxy, carboxyl, carboalkoxy, or amino groups. Examples of 2'-O-substituted ribonucleosides or 2'-O-substituted-arabinosides include, without limitation 2'-O-methylribonucleosides or 2'-O-methylarabinosides and 2'-O-methoxyethylribonucleosides or 2'-O-methoxyethylarabinosides.

The term "2'-substituted ribonucleoside" or "2'-substituted arabinoside" also includes ribonucleosides or arabinonucleosides in which the 2'-hydroxyl group is replaced with a lower alkyl group containing 1-6 saturated or unsaturated carbon atoms, or with an amino or halo group. Examples of such 2'-substituted ribonucleosides or 2'-substituted arabinosides include, without limitation, 2'-amino, 2'-fluoro, 2'-allyl, and 2'-propargyl ribonucleosides or arabinosides.

The term "oligonucleotide" includes hybrid and chimeric oligonucleotides. A "chimeric oligonucleotide" is an oligonucleotide having more than one type of internucleoside linkage. One preferred example of such a chimeric oligonucleotide is a chimeric oligonucleotide comprising a phosphorothioate, phosphodiester or phosphorodithioate region and non-ionic linkages such as alkylphosphonate or alkylphosphonothioate linkages (see *e.g.*, Pederson et al. U.S. Patent Nos. 5,635,377 and 5,366,878).

A "hybrid oligonucleotide" is an oligonucleotide having more than one type of nucleoside. One preferred example of such a hybrid oligonucleotide comprises a ribonucleotide or 2'-substituted ribonucleotide region, and a deoxyribonucleotide region (see, *e.g.*, Metelev and Agrawal, U.S. Patent No. 5,652,355, 6,346,614 and 6,143,881).

For purposes of the invention, the term "immunostimulatory oligonucleotide" refers to an oligonucleotide as described above that induces an immune response when administered to a vertebrate, such as a fish, fowl, or mammal. As used herein, the term "mammal" includes, without limitation rats, mice, cats, dogs, horses, cattle, cows, pigs, rabbits, non-human primates, and humans. Useful immunostimulatory oligonucleotides can be found described in Agrawal et al., WO 98/49288, published November 5,1998; WO 01/12804, published February 22, 2001; WO 01/55370, published August 2,2001; PCT/US01/13682, filed April 30, 2001; and PCT/US01/30137, filed September 26, 2001. Preferably, the immunostimulatory oligonucleotide comprises at least one phosphodiester, phosphorothioate, or phosphorodithioate internucleoside linkage.

In some embodiments, the immunostimulatory oligonucleotide comprises an immunostimulatory dinucleotide of formula 5'-Pyr-Pur-3', wherein Pyr is a natural or synthetic pyrimidine nucleoside and Pur is a natural or synthetic purine nucleoside. In some preferred embodiments, the immunostimulatory oligonucleotide comprises an immunostimulatory dinucleotide of formula 5'-Pur*-Pur-3', wherein Pur* is a synthetic purine nucleoside and Pur is a natural or synthetic purine nucleoside. In various places the dinucleotide is expressed as RpG, C*pG or YZ, in which case respectively, R, C*, or Y represents a synthetic purine. A particularly preferred synthetic purine is 2-oxo-7-deaza-8-methyl-purine. When this synthetic purine is in the Pur* position of the dinucleotide, species-specificity (sequence dependence) of the immunostimulatory effect is overcome and cytokine profile is improved. As used herein, the term "pyrimidine nucleoside" refers to a nucleoside wherein the base component of the nucleoside is a monocyclic nucleobase. Similarly, the term "purine nucleoside" refers to a nucleoside wherein the base component of the nucleoside is a bicyclic nucleobase. For purposes of the invention, a "synthetic" pyrimidine or purine nucleoside includes a non-naturally occurring pyrimidine or purine base, a non-naturally occurring sugar moiety, or a combination thereof.

### Preferred pyrimidine nucleosides have the structure (I):

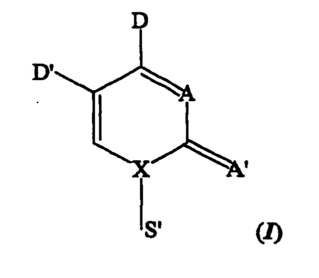

wherein:
D is a hydrogen bond donor;
D' is selected from the group consisting of hydrogen, hydrogen bond donor, hydrogen bond acceptor, hydrophilic group, hydrophobic group, electron withdrawing group and electron donating group;
A is a hydrogen bond acceptor or a hydrophilic group;
A' is selected from the group consisting of hydrogen bond acceptor, hydrophilic group, hydrophobic group, electron withdrawing group and electron donating group;
X is carbon or nitrogen; and
S' is a pentose or hexose sugar ring, or a non-natuxally occurring sugar.

Preferably, the sugar ring is derivatized with a phosphate moiety, modified phosphate moiety, or other linker moiety suitable for linking the pyrimidine nucleoside to another nucleoside or nucleoside analog.

Preferred hydrogen bond donors include, without limitation, -NH-, -NH₂, -SH and -OH. Preferred hydrogen bond acceptors include, without limitation, C=O, C=S, and the ring nitrogen atoms of an aromatic heterocycle, e.g., N3 of cytosine.

In some embodiments, the base moiety in (***I***) is a non-naturally occurring pyrimidine base. Examples of preferred non-naturally occurring pyrimidine bases include, without limitation, 5-hydroxycytosine, 5-hydroxymethylcytosine, N4-alkylcytosine, preferably N4-ethylcytosine, and 4-thiouracil. However, in some embodiments 5-bromocytosine is specifically excluded.

In some embodiments, the sugar moiety S' in (***I***) is a non-naturally occurring sugar moiety. For purposes of the present invention, a "naturally occurring sugar moiety" is a sugar moiety that occurs naturally as part of nucleic acid, e.g., ribose and 2'-deoxyribose, and a "non-naturally occurring sugar moiety" is any sugar that does not occur naturally as part of a nucleic acid, but which can be used in the backbone for an oligonucleotide, e.g, hexose. Arabinose and arabinose derivatives are examples of preferred sugar moieties.

### Preferred purine nucleoside analogs have the structure (II):

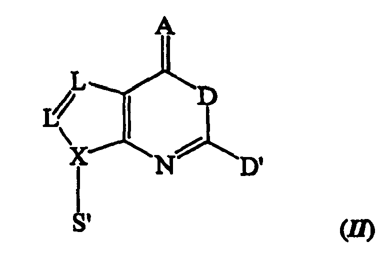

wherein:
D is a hydrogen bond donor;
D' is selected from the group consisting of hydrogen, hydrogen bond donor, and hydrophilic group;
A is a hydrogen bond acceptor or a hydrophilic group;
X is carbon or nitrogen;
each L is independently an atom selected from the group consisting of C, O, N and S; and
S' is a pentose or hexose sugar ring, or a non-naturally occurring sugar.

Preferably, the sugar ring is derivatized with a phosphate moiety, modified phosphate moiety, or other linker moiety suitable for linking the pyrimidine nucleoside to another nucleoside or nucleoside analog.

Preferred hydrogen bond donors include, without limitation, -NH-, NH₂, -SH and -OH. Preferred hydrogen bond acceptors include, without limitation, C=O, C=S, -NO₂ and the ring nitrogen atoms of an aromatic heterocycle, e.g., N1 of guanine.

In some embodiments, the base moiety in (***II***) is a non-naturally occurring purine base. Examples of preferred non-naturally occurring purine bases include, without limitation, 2-amino-6-thiopurine and 2-amino-6-oxo-7-deazapurine. In some embodiments, the sugar moiety S' in (***II***) is a naturally occurring sugar moiety, as described above for structure (***I***).

In preferred embodiments, the immunostimulatory dinucleotide is selected from the group consisting of CpG, C*pG, CpG*, and C*pG*, wherein the base of C is cytosine, the base of C* is 2'-thymine, 5-hydroxycytosine, N4-alkyl-cytosine, 4-thiouracil or other non-natural pyrimidine, or 2-oxo-7-deaza-8-methylpurine, wherein when the base is 2-oxo-7-deaza-8-methyl-purine, it is preferably covalently bound to the 1'-position of a pentose via the 1 position of the base; the base of G is guanosine, the base of G* is 2-amino-6-oxo-7-deazapurine, 2-oxo-7-deaza-8-methylpurine, 6-thioguanine, 6-oxopurine, or other non-natural purine nucleoside, and p is an internucleoside linkage selected from the group consisting of phosphodiester, phosphorothioate, and phosphorodithioate. In certain preferred embodiments, the immunostimulatory dinucleotide is not CpG.

Preferred immunostimulatory moieties further include nucleosides having sugar modifications, including, without limitation, 2'-substituted pentose sugars including, without limitation, 2'-O-methylribose, 2'-O-methoxyethylribose, 2'-O-propargylribose, and 2'-deoxy-2'-fluororibose; 3'-substituted pentose sugars, including, without limitation, 3'-O-methylribose; 1',2'-dideoxyribose; arabinose; substituted arabinose sugars, including, without limitation, 1'-methylarabinose, 3'-hydroxymethylarabinose, 4'-hydroxymethylarabinose, 3'-hydroxyarabinose and 2'-substituted arabinose sugars; hexose sugars, including, without limitation, 1,5-anhydrohexitol; and alpha-anomers. In embodiments in which the modified sugar is a 3'-deoxyribonucleoside or a 3'-O-substituted ribonucleoside, the immunostimulatory moiety is attached to the adjacent nucleoside by way of a 2'-5' internucleoside linkage.

Preferred immunostimulatory moieties further include oligonucleotides having other carbohydrate backbone modifications and replacements, including peptide nucleic acids (PNA), peptide nucleic acids with phosphate groups (PHONA), locked nucleic acids (LNA), morpholino backbone oligonucleotides, and oligonucleotides having backbone linker sections having a length of from about 2 angstroms to about 200 angstroms, including without limitation, alkyl linkers or amino linkers. The alkyl linker may be branched or unbranched, substituted or unsubstituted, and chirally pure or a racemic mixture. Most preferably, such alkyl linkers have from about 2 to about 18 carbon atoms. In some preferred embodiments such alkyl linkers have from about 3 to about 9 carbon atoms. Some alkyl linkers include one or more functional groups selected from the group consisting of hydroxy, amino, thiol, thioether, ether, amide, thioamide, ester, urea, and thioether. Some such functionalized alkyl linkers are poly(ethylene glycol) linkers of formula -O-(CH₂-CH₂-O-)*ₙ* (*n* = 1-9). Some other functionalized alkyl linkers are peptides or amino acids.

Preferred immunostimulatory moieties further include DNA isoforms, including, without limitation, β-L-deoxyribonucleosides and α-deoxyribonucleosides. Preferred immunostimulatory moieties incorporate 3' modifications, and further include nucleosides having unnatural internucleoside linkage positions, including, without limitation, 2'-5', 2'-2', 3'-3' and 5'-5' linkages.

Preferred immunostimulatory moieties further include nucleosides having modified heterocyclic bases, including, without limitation, 5-hydroxycytosine, 5-hydroxymethylcytosine, N4-alkylcytosine, preferably N4-ethylcytosine, 4-thiouracil, 6-thioguanine, 7-deazaguanine, inosine, nitropyrrole, C5-propynylpyrimidine, and diaminopurines, including, without limitation, 2,6-diaminopurine.

The immunostimulatory oligonucleotides according to the invention comprise at least two oligonucleotides linked at their 3' ends or internucleoside linkage or a functionalized nucleobase or sugar via a non-nucleotidic glycerol linker.

The term "non-nucleotidic glycerol linker" is not meant to refer to an internucleoside linkage, as described above, e.g., a phosphodiester, phosphorothioate, or phosphorodithioate functional group, that directly connects the 3'-hydroxyl groups of two nucleosides. For purposes of this invention, such a direct 3'-3' linkage (no linker involved) is considered to be a "nucleotidic linkage."

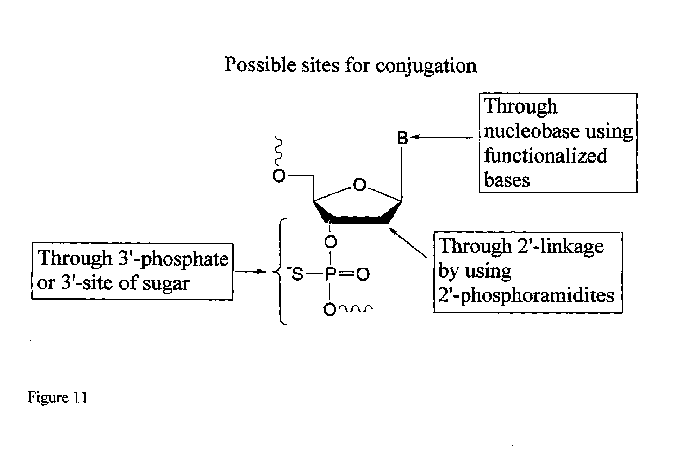

As a non-limiting example, the glycerol linker may be attached to any suitable position on the nucleoside, as illustrated in Figure 11. In some preferred embodiments, the glycerol linker is attached to the 3'-hydroxyl. In such embodiments, a hydroxyl functional group of the glycerol is preferably attached to the 3'-hydroxyl by means of a phosphodiester, phosphorothioate, phosphorodithioate or non-phosphate-based linkages.

The linker glycerol has three hydroxyl groups to which oligonucleotides may be covalently attached. Some immunostimulatory oligonucleotides therefore, may comprise more than two oligonucleotides linked at their 3' ends to glycerol.

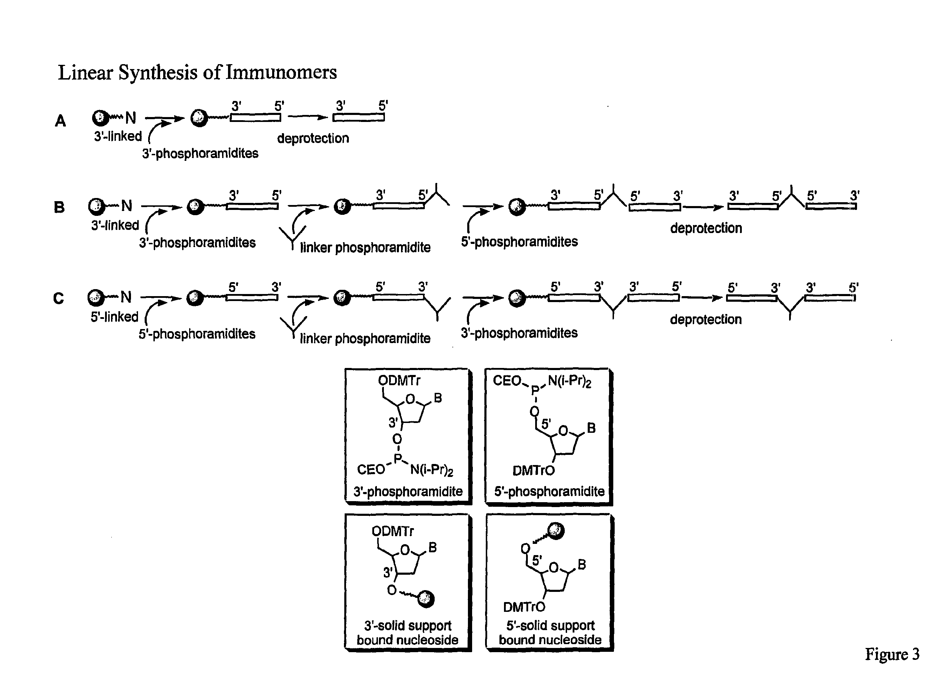

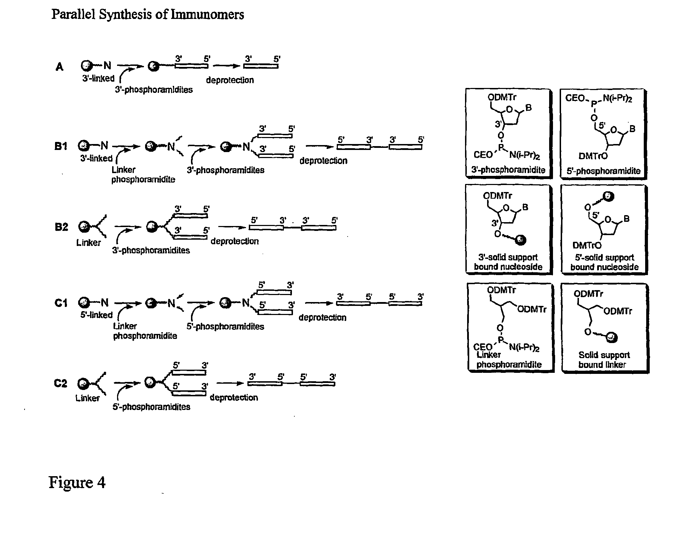

The immunostimulatory oligonucleotides of the invention may conveniently be synthesized using an automated synthesizer and phosphoramidite approach as schematically depicted in Figures 3 and 4, and further described in the Examples. In some embodiments, the immunostimulatory oligonucleotides are synthesized by a linear synthesis approach (see Figure 3). As used herein, the term "linear synthesis" refers to a synthesis that starts at one end of the immunostimulatory oligonucleotide and progresses linearly to the other end. Linear synthesis permits incorporation of either identical or un-identical (in terms of length, base composition and/or chemical modifications incorporated) monomeric units into the immunostimulatory oligonucleotides.

An alternative mode of synthesis is "parallel synthesis", in which synthesis proceeds outward from a central linker moiety (see Figure 4). A solid support attached linker can be used for parallel synthesis, as is described in U.S. Patent No. 5,912,332. Alternatively, a universal solid support (such as phosphate attached controlled pore glass) support can be used.

Parallel synthesis of immunostimulatory oligonucleotides has several advantages over linear synthesis: (1) parallel synthesis permits the incorporation of identical monomeric units; (2) unlike in linear synthesis, both (or all) the monomeric units are synthesized at the same time, thereby the number of synthetic steps and the time required for the synthesis is the same as that of a monomeric unit; and (3) the reduction in synthetic steps improves purity and yield of the final immunostimulatory oligonucleotide product.

At the end of the synthesis by either linear synthesis or parallel synthesis protocols, the immunostimulatory oligonucleotides may conveniently be deprotected with concentrated ammonia solution or as recommended by the phosphoramidite supplier, if a modified nucleoside is incorporated. The product immunostimulatory oligonucleotide is preferably purified by reversed phase HPLC, detritylated, desalted and dialyzed.

Table 1 shows representative immunostimulatory oligonucleotides according to the invention.

**Table 1. Examples of Immunostimulatory Oligonncleotides Sequences**

| SEQ ID NO. | Sequences and Modification |
|---|---|
| 1 | 5'-TC**G₁**AAC**G₁**TTC**G₁**-X-**G₁**CTT**G₁**CAA**G₁**CT-5' (control) |
| 2 | 5'-TCAGTC**G₂**TTAG-X-GATT**G₂**CTGACT-5' |
| 3 | 5'-TC**G₂**TC**G₂**TTAGA-X-AGATT**G₂**CT**G₂**CT-5' (control) |
| 4 | 5'-CAGTC**G₂**TTCAG-X-GACTT**G₂**CTGAC-5' (control) |
| 5 | 5'-TCAGTC**G₁**TTAG-X-GATT**G₁**CTGACT-5' |
| 6 | 5'-TC**G₁**TC**G₁**TTAGA-X-AGATT**G₁**CT**G₁**CT-5' (control) |
| 7 | 5'-CAGTC**G₁**TTCAG-X-GACTT**G₁**CTGAC-5' (control) |
| 8 | 5'-ACACACCAACT-TCAACCACACA-5' (control) |

| | |
|---|---|
| G₁ = 2'-deoxy-7-deazaguanosine; G₂ = araguanosine; X = glycerol linker | |

The invention also provides immunostimulatory oligonucleotide conjugates comprising an immunostimulatory oligonucleotide, as described above, and an antigen conjugated to the immunostimulatory oligonucleotide at a position other than the accessible 5' end. In some embodiments, the glycerol linker comprises an antigen, which is conjugated to the oligonucleotide. In some other embodiments, the antigen is conjugated to the oligonucleotide at a position other than its 3' end. In some embodiments, the antigen produces a vaccine effect.

The antigen is preferably selected from the group consisting of antigens associated with a pathogen, antigens associated with a cancer, antigens associated with an auto-immune disorder, and antigens associated with other diseases such as, but not limited to, veterinary or pediatric diseases. For purposes of the invention, the term "associated with" means that the antigen is present when the pathogen, cancer, auto-immune disorder, food allergy, respiratory allergy, asthma or other disease is present, but either is not present, or is present in reduced amounts, when the pathogen, cancer, auto-immune disorder, food allergy, respiratory allergy, or disease is absent

The immunostimulatory oligonucleotide is covalently linked to the antigen, or it is otherwise operatively associated with the antigen. As used herein, the term "operatively associated with" refers to any association that maintains the activity of both immunostimulatory oligonucleotide and antigen. Nonlimiting examples of such operative associations include being part of the same liposome or other such delivery vehicle or reagent. In embodiments wherein the immunostimulatory oligonucleotide is covalently linked to the antigen, such covalent linkage preferably is at any position on the immunostimulatory oligonucleotide other than an accessible 5' end of an immunostimulatory oligonucleotide. For example, the antigen may be attached at an internucleoside linkage or may be attached to the non-nucleotidic linker. Alternatively, the antigen may itself be the non-nucleotidic linker.

The invention also provides pharmaceutical formulations comprising an immnnostimulatory oligonucleotide or immunostimulatory oligonucleotide conjugate according to the invention and a physiologically acceptable carrier. As used herein, the term "physiologically acceptable" refers to a material that does not interfere with the effectiveness of the immunostimulatory oligonucleotide and is compatible with a biological system such as a cell, cell culture, tissue, or organism. Preferably, the biological system is a living organism, such as a vertebrate.

As used herein, the term "carrier" encompasses any excipient, diluent, filler, salt, buffer, stabilizer, solubilizer, lipid, or other material well known in the art for use in pharmaceutical formulations. It will be understood that the characteristics of the carrier, excipient, or diluent will depend on the route of administration for a particular application. The preparation of pharmaceutically acceptable formulations containing these materials is described in, *e.g.,* Remington's Pharmaceutical Sciences, 18th Edition, ed. A. Gennaro, Mack Publishing Co., Easton, PA, 1990.

The invention also provides an immunostimulatory oligonucleotide or imanunostimulatory oligonucleotide conjugate according to the invention for use in a method for generating an immune response in a vertebrate, such method comprising administering the oligonucleotide or conjugate to the vertebrate.

In some embodiments, the vertebrate is a mammal. For purposes of this invention, the term "mammal" is expressly intended to include humans. In preferred embodiments, the immunostimulatory oligonucleotide or immunostimulatory oligonucleotide conjugate is administered to a vertebrate in need of immunostimulation.

In the methods administration of immunostimutatory oligonucleotide or immunostimulatory oligonucleotide conjugate can be by any suitable route, including, without limitation, parenteral, oral, sublingual, transdermal, topical, intranasal, aerosol, intraocular, intratracheal, intrarectal vaginal, by gene gun, dermal patch or in eye drop or mouthwash form. Administration of the therapeutic compositions of immunostimulatory oligonucleotides can be carried out using known procedures at dosages and for periods of time effective to reduce symptoms or surrogate markers of the disease. When administered systemically, the therapeutic composition is preferably administered at a sufficient dosage to attain a blood level of immunostimulatory oligonucleotide from about 0.0001 micromolar to about 10 micromolar. For localized administration, much lower concentrations than this may be effective, and much higher concentrations may be tolerated. Preferably, a total dosage of immunostimulatory oligonucleotide ranges from about 0.001 mg per patient per day to about 200 mg per kg body weight per day. It may be desirable to administer simultaneously, or sequentially a therapeutically effective amount of one or more of the therapeutic compositions of the invention to an individual as a single treatment episode.

In certain preferred embodiments, immunostimulatory oligonucleotide or immunostimulatory oligonucleotide conjugate according to the invention are administered in combination with vaccines, antibodies, cytotoxic agents, allergens, antibiotics, antisense oligonucleotides, peptides, proteins, gene therapy vectors, DNA vaccines and/or adjuvants to enhance the specificity or magnitude of the immune response. In these embodiments, the immunostimulatory oligonucleotides of the invention can variously act as adjuvants and/or produce direct immunostimulatory effects.

Either the immunostimulatory oligonucleotide or immunostimulatory oligonucleotide conjugate or the vaccine, or both, may optionally be linked to an immunogenic protein, such as keyhole limpet hemocyanin (KLH), cholera toxin B subunit, or any other immunogenic carrier protein. Any of the plethora of adjuvants may be used including, without limitation, Freund's complete adjuvant, KLH, monophosphoryl lipid A (MPL), alum, and saponins, including QS-21, imiquimod, R848, or combinations thereof.

For purposes of this aspect of the invention, the term "in combination with" means in the course of treating the same disease in the same patient, and includes administering the immunostimulatory oligonucleotide and/or the vaccine and/or the adjuvant in any order, including simultaneous administration, as well as temporally spaced order of up to several days part Such combination treatment may also include more than a single administration of the immunostimulatory oligonucleotide, and/or independently the vaccine, and/or independently the adjuvant. The administration of the immunostimulatory oligonucleotide and/or vaccine and/or adjuvant may be by the same or different routes.

The methods according to this aspect are useful for model studies of the immune system. The methods are also useful for the prophylactic or therapeutic treatment of human or animal disease. For example, the methods are useful for pediatric and veterinary vaccine applications.

The invention also provides an immunostimulatory oligonucleotide or immunostimulatory oligonucleotide conjugate according to the invention for use in a method for therapeutically treating a patient having a disease or disorder, such method comprising administering the oligonucleotide or conjugate to the patient. In various embodiments, the disease or disorder to be treated is cancer, an autoimmune disorder, airway inflammation, inflammatory disorders, allergy, asthma or a disease caused by a pathogen. Pathogens include bacteria, parasites, fungi, viruses, viroids and prions. Administration is carried out as described above.

For purposes of the invention, the term "allergy" includes, without limitation, food allergies and respiratory allergies. The term "airway inflammation" includes, without limitation, asthma. As used herein, the term "autoimmune disorder" refers to disorders in which "self' proteins undergo attack by the immune system. Such term includes autoimmune asthma.

In any of the methods, the immunostimulatory oligonucleotide or immunostimulatory oligonucleotide conjugate can be administered in combination with any other agent useful for treating the disease or condition that does not diminish the immunostimulatory effect of the immunostunulatory oligonucleotide. For example, in the treatment of cancer, it is contemplated that the immunostimulatory oligonucleotide or immunostimulatory oligonucleotide conjugate may be administered in combination with a chemotherapeutic compound.

The examples below are intended to further illustrate certain preferred embodiments of the invention, and are not intended to limit the scope of the invention.

### EXAMPLES

### Example 1: Synthesis of Oligonncleotides Containing immunostimulatory Moieties

Oligonucleotides were synthesized on a 1 µmol to 0.1 mM scale using an automated DNA synthesizer (OligoPilot II, AKTA, (Amersham) and/or Expedite 8909 (Applied Biosystems)), following the linear synthesis or parallel synthesis procedures outlined in Figures 3 and 4.

5'-DMT dA, dG, dC and T phosphoramidites were purchased from Proligo (Boulder, CO). 5'-DMT 7-deaza-dG and araG phosphoramidites were obtained from Chemgenes (Wilmington, MA). DiDMT-glycerol linker solid support was obtained from Chemgenes. 1-(2'-deoxy-β-D-ribofuranosyl)-2-oxo-7-deaza-8-methyl-purine amidite was obtained from Glen Research (Sterling, VA), 2'-O-methylribonuncleoside amidites were obtained from Promega (Obispo, CA). All oligonucleotides were phosphorothioate backbone modified.

All nucleoside phosphoramidites were characterized by ³¹P and ¹H NMR spectra. Modified nucleosides were incorporated at specific sites using normal coupling cycles recommended by the supplier. After synthesis, oligonucleotides were deprotected using concentrated ammonium hydroxide and purified by reverse phase HPLC, detritylation, followed by dialysis. Purified oligonucleotides as sodium salt form were lyophilized prior to use. Purity was tested by CGE and MALDI-TOF MS. Endotoxin levels were determined by LAL test and were below 1.0 EU/mg.

### Example 2: Activation of TLRs

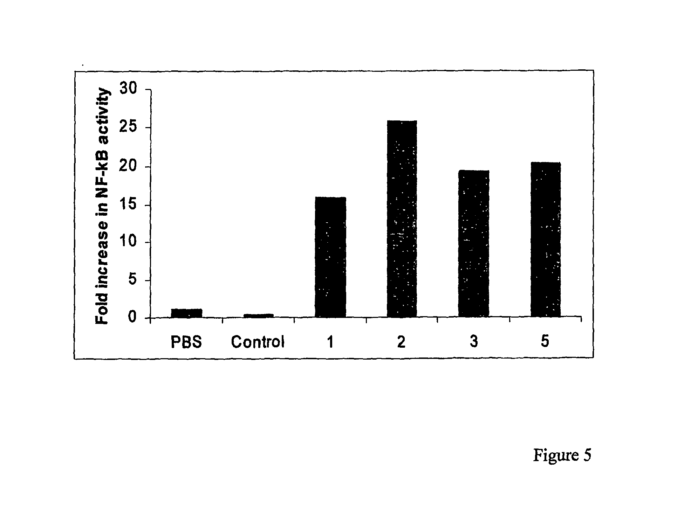

HEK293/mTLR9 cells (Invivogen, San Diego, CA) were cultured in 48-well plates in 250 µl/well DMEM supplemented with 10% heat-inactivated FBS in a 5% CO₂ incubator. At 80% confluence, cultures were transiently transformed with 400 ng/ml of Seap reporter plasmid (pNifty2-Seap) (San Diego CA) in the presence of 4 µl/ml of Lipofectannine (Invitrogen, CA) in culture medium. Plasmid DNA and Lipofectamine were diluted separately in serum-free medium and incubated at room temperature for 5 minutes. After incubation, the diluted DNA and Lipofectamine were mixed and the mixtures were incubated at room temperature for 20 minutes. 25 µl of the DNA/Lipofectamine mixture containing 100 ng plasmid DNA and 1 µl of Lipofectamine was added to each well of the cell culture plate, and the cultures were continued for 4 hours. After transformation, medium was replaced with fresh culture medium, and stimulating and inhibitory oligos were added to the cultures as the following:
Stimulating oligo at 0.5 µg/ml, plus inhibitory oligo at 0, 0.25, 0.5, 2.0, 5.0 µg/ml;
Cultures were continued for 18 hours. At the end of oligo treatment, 30 µl of culture supernatant was taken from each treatment and used for SEAP assay. Manufacturer's protocol (Invivogen) was followed for the assay. Signals were detected by a plate reader at 405 nm. All OD readings were norrmalized by medium control (treatment OD - medium OD), and NF-κB activity was expressed as fold of PBS control (normalized treatment/normalixed PBS). % Activity was calculated by taking stimulating oligo x 0.5 µg group as 100%, and all other treatment groups as percentages of that of stimulating oligo x 0.5 µg group. The results are shown in Figure 5.

### Example 3: IFN-α induction in human pDC

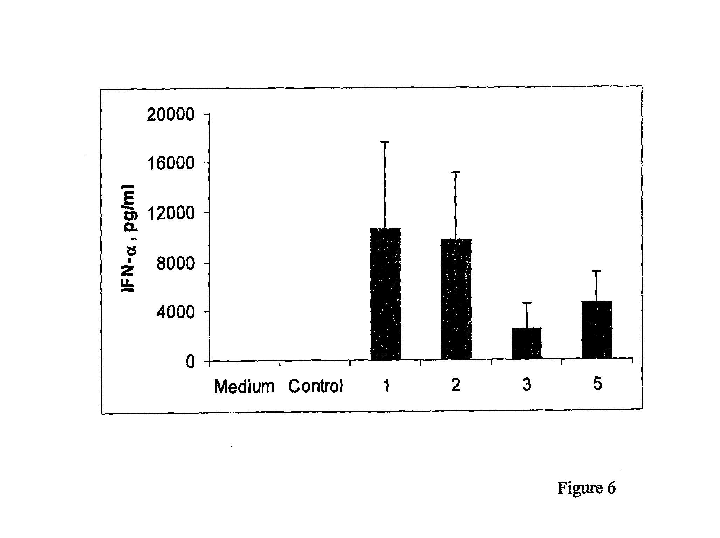

Peripheral blood mononuclear cells (PBMCs) from freshly drawn healthy volunteer blood (CBR Laboratories, Boston, MA) were isolated by Ficoll density gradient centrifugation method (Histopaque-1077, Sigma). pDCs were isolated from PBMCs by positive selection using the BDCA4 cell isolation kits (Miltenyi Biotec) according to the manufacturer's instructions. pDCs were plated in 96-well dishes using 1X10⁶ cells/ml. The IMOs dissolved in DPBS (pH 7.4; Mediatech) were added to a final concentration of 10.0 µg/ml to the cell cultures. The cells were then incubated at 37 °C for 24 hr and the supernatants were collected for ELISA assays. The experiments were performed in triplicate wells. The levels of IFN-α were measured by sandwich ELISA. The required reagents, including cytokine antibodies and standards, were purchased from PharMingen. The results are shown in Figure 6.

### Example 4: Cytokine induction by IMO in human PBMCs

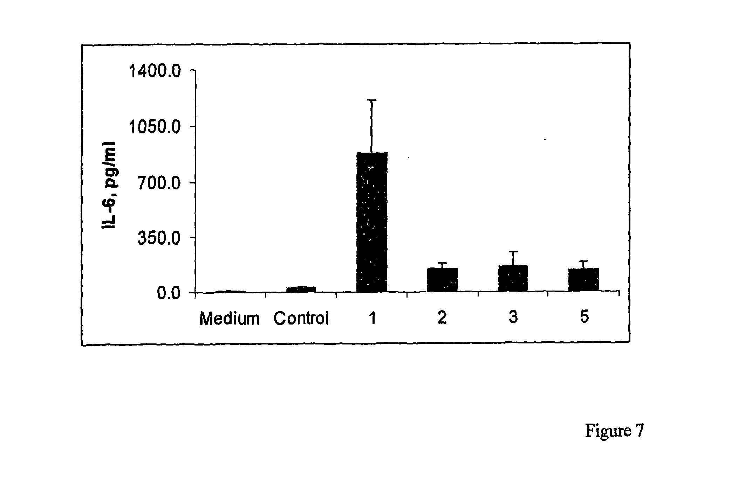

Human PBMCs were plated in 48-well plates using 5X10⁶ cells/ml. The IMOs dissolved in DPBS (pH 7.4; Mediatech) were added to a final concentration of 10.0 µg/ml to the cell cultures. The cells were then incubated at 37 °C for 24 hr and the supernatants were collected for ELISA assays. The experiments were performed in triplicate wells. The levels of IL-6 were measured by sandwich ELISA. The required reagents, including cytokine antibodies and standards, were purchased from PharMingen. The results are shown in Figure 7.

### Example 5: Human B-Cell Proliferation

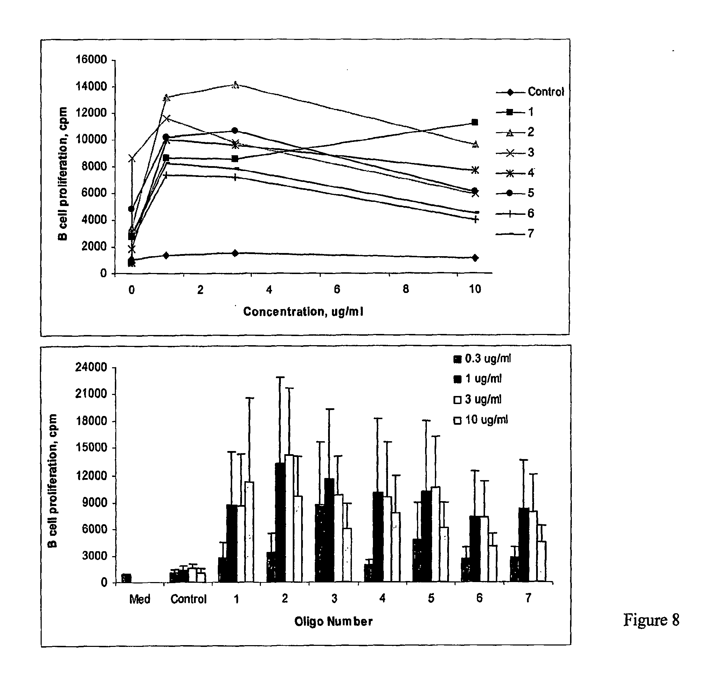

The culture medium used for the assay consisted of RPMI 1640 medium supplemented with 1.5 mM glutamine, 1 mM sodium pyruvate, 0.1 mM non-essential amino acids, 50 µM 2-mercaptoethanol, 100 IU/ml penicillin-streptomycin mix and 10% heat-inactivated fetal bovine serum. A total of 0.5 X 10⁶ B cells per ml (i.e.1 X 10⁵ /200 µl/well) were stimulated in 96 well flat bottom plates with different concentrations of test oligonucleotides in triplicate for a total period of 72 hours. After 66 h, cells were pulsed with 0.75 µCi of [³H]-thymidine (1Ci = 37 GBq; Pekin Elmer Life Sciences) in 20 µl RPMI 1640 medium (no serum) per well and harvested 8h later. The plates were then harvested using a cell harvester and radioactive incorporation was determined using standard liquid scintillation technique. The results, shown in Figure 8, are expressed either as mean cpm +/-SD or as proliferation index (cpm treated group/cpm medium control).

### Example 6: Cytokine induction in mouse spleen cell cultures

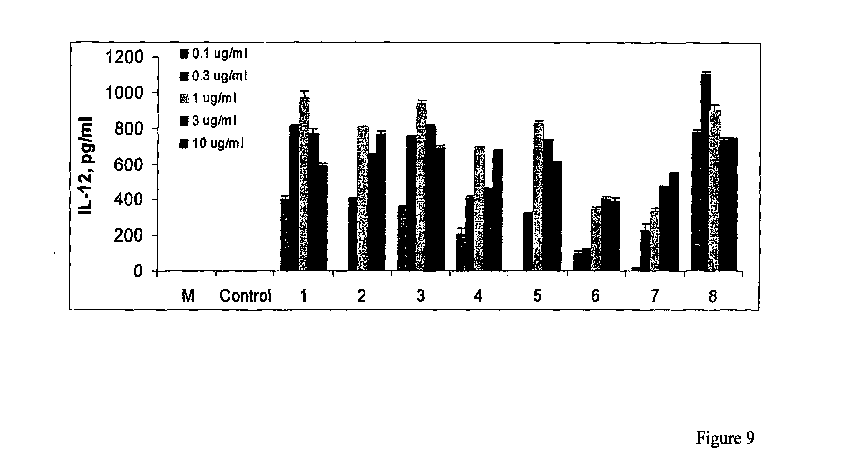

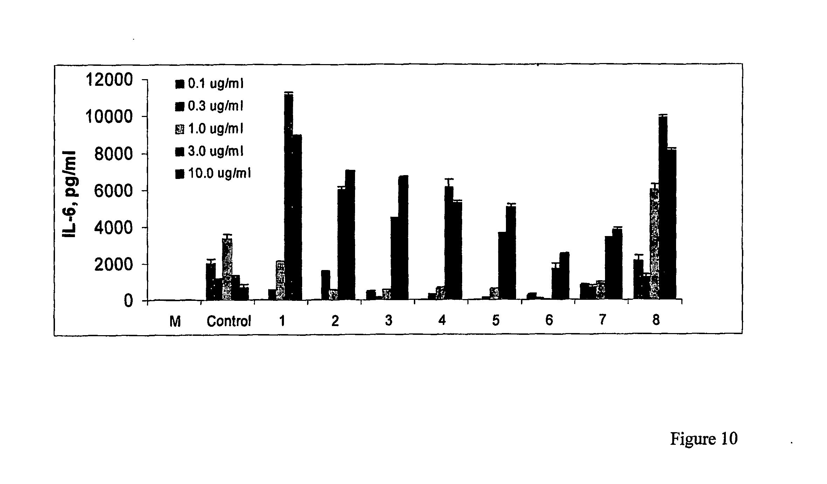

Spleen cells from 4-8 week old BALB/c or C57BL/6 mice were prepared and cultured in RPMI complete medium. Mouse spleen cells were plated in 24-well dishes at 5 X 106 cells /ml. IMOs dissolved in TE bluffer. (10 mM Tris-HCL, pH 7.5, 1 mM EDTA) were added to a final concentration of 0.03, 0.1, 1.0, 3.0 or 10 µg/ml to the cell cultures. The cells were then incubated at 37 °C for 24 hr and the supernatants were collected for ELISA assays. IL-12 and IL-6 levels in supernatants were measured by sandwich ELISA. The required reagents including cytokine antibodies and standards were purchased from BD Pharmingen. Streptavidin-Peroxidase and substrate were from KPL. The results are shown in Figures 9 and 10.

### EQUIVALENTS

While the foregoing invention has been described in some detail for purposes of clarity and understanding, it will be appreciated by one skilled in the art from a reading of this disclosure that various changes in form and detail can be made without departing from the true scope of the invention and appended claims.

## Claims

1. An immunostimulatory oligonucleotide having a structure from the group of 5'-TCAGTCG₂TTAG-X-GATTG₂CTGACT-5' and 5'-TCAGTCG₁TTAG-X-GATTG₁CTGACT-5';
wherein G₁ = 2'-deoxy-7-deazaguanosine; G₂ = arabinoguanosine; and X = glycerol linker.

2. A composition comprising the oligonucleotide according to Claim 1.

3. The composition according to Claim 2, further comprising an antibody, antisense oligonucleotide, protein, antigen, allergen, chemotherapeutic agent or adjuvant.

4. A pharmaceutical formulation comprising the oligonucleotide according to Claim 1 and a physiologically acceptable carrier.

5. The pharmaceutical formulation according to Claim 4, further comprising an antibody, antisense oligonucleotide, protein, antigen, allergen, chemotherapeutic agent or adjuvant.

6. An immunostimulatory oligonucleotide having a structure from the group of 5'-TCAGTCG₂TTAG-X-GATTG₂CTGACT-5' and 5'-TCAGTCG₁TTAG-X-GATTG₁CTGACT-5'; wherein G₁ = 2'-deoxy-7-deazaguanosine; G₂ = arabinoguanosine; and X = glycerol linker for use in generating an immune response in a vertebrate.

7. The oligonucleotide according to Claim 6, wherein the route of administration is selected from parenteral, oral, sublingual, transdermal, topical, intranasal, aerosol, intraocular, intratracheal, intrarectal, vaginal, gene gun, dermal patch, eye drop and mouthwash.

8. An immunostimulatory oligonucleotide having a structure from the group of 5'-TCAGTCG₂TTAG-X-GATTG₂CTGACT-5' and TCAGTCG₁TTAG-X-GATTG₁CTGACT-5';
wherein G₁ = 2'-deoxy-7-deazaguanosine; G₂ = arabinoguanosine; and X = glycerol linker for use in therapeutically treating a vertebrate having cancer, an autoimmune disorder, airway inflammation, inflammatory disorders, skin disorders, allergy, asthma or a disease caused by a pathogen.

9. The oligonucleotide according to Claim 8, wherein the route of administration is selected from parenteral, oral, sublingual, transdermal, topical, intranasal, aerosol, intraocular, intratracheal, intrarectal, vaginal, gene gun, dermal patch, eye drop and mouthwash.

10. An immunostimulatory oligonucleotide having a structure from the group of 5'-TCAGTCG₂TTAG-X-GATTG₂CTGACT-5' and 5'-TCAGTCG₁TTAG-X-GATTG₁CTGACT-5'; wherein G₁ = 2'-deoxy-7-deazaguanosine; G₂ = arabinoguanosine; and X = glycerol linker for use in preventing cancer, an autoimmune disorder, airway inflammation, inflammatory disorders, skin disorders, allergy, asthma or a disease caused by a pathogen in a vertebrate.

11. The oligonucleotide according to Claim 10, wherein the route of administration is selected from parenteral, oral, sublingual, transdermal, topical, intranasal, aerosol, intraocular, intratracheal, intrarectal, vaginal, gene gun, dermal patch, eye drop and mouthwash.

12. The oligonucleotide according to Claim 6, 8 or 10, which is to be administered together with an antibody, antisense oligonucleotide, protein, antigen, allergen, chemotherapeutic agent or adjuvant.

13. Use of an immunostimulatory oligonucleotide having a structure from the group of 5'-TCAGTCG₂TTAG-X-GATTG₂CTGACT-5' and 5'-TCAGTCG₁TTAG-X-GATTG₁CTGACT-5' wherein G₁ = 2'-deoxy-7-deazaguanosine; G₂ = arabinoguanosine; and X = glycerol linker for the preparation of a pharmaceutical formulation for generating an immune response in a vertebrate.

14. Use of an immunostimulatory oligonucleotide having a structure from the group of 5'-TCAGTCG₂TTAG-X-GATTG₂CTGACT-5' and 5'-TCAGTCG₁TTAG-X-GATTG₁CTGACT-5'; wherein G₁ = 2'-deoxy-7-deazaguanosine; G₂ = arabinoguanosine; and X = glycerol linker for the preparation of a pharmaceutical formulation for therapeutically treating a vertebrate having cancer, an autoimmune disorder, airway inflammation, inflammatory disorders, skin disorders, allergy, asthma or a disease caused by a pathogen.

15. Use of an immunostimulatory oligonucleotide having a structure from the group of 5'-TCAGTCG₂TTAG-X-GATTG₂CTGACT-5' and 5'-TCAGTCG₁TTAG-X-GATTG₁CTGACT-5';
wherein G₁ = 2'-deoxy-7-deazaguanosine; G₂ = arabinoguanosine; and X = glycerol linker for the preparation of a pharmaceutical formulation for preventing cancer, an autoimmune disorder, airway inflammation, inflammatory disorders, skin disorders, allergy, asthma or a disease caused by a pathogen in a vertebrate.

16. The use according to Claim 13, 14 or 15, wherein the immunostimulatory oligonucleotide is to be administered together with an antibody, antisense oligonucleotide, protein, antigen, allergen, chemotherapeutic agent or adjuvant.

## Patentansprüche

1. Immunstimulierendes Oligonukleotid mit einer Struktur aus der Gruppe 5'-TCAGTCG₂TTAG-X-GATTG₂CTGACT-5' und 5'-TCAGTCG₁TTAG-X-GATTG₁CTGACT-5', worin G₁ = 2'-Desoxy-7-deazaguanosin, G₂ = Arabinoguanosin und X = Glycerinlinker.

2. Zusammensetzung, die das Oligonukleotid gemäß Anspruch 1 umfasst.

3. Zusammensetzung gemäß Anspruch 2, die ferner einen Antikörper, ein Antisense-Oligonukleotid, ein Protein, ein Antigen, ein Allergen, ein Chemotherapeutikum oder ein Adjuvanz umfasst.

4. Pharmazeutische Formulierung, die das Oligonukleotid gemäß Anspruch 1 und einen physiologisch verträglichen Träger umfasst.

5. Pharmazeutische Formulierung gemäß Anspruch 4, die ferner einen Antikörper, ein Antisense-Oligonukleotid, ein Protein, ein Antigen, ein Allergen, ein Chemotherapeutikum oder ein Adjuvanz umfasst.

6. Immunstimulierendes Oligonukleotid mit einer Struktur aus der Gruppe 5'-TCAGTCG₂TTAG-X-GATTG₂CTGACT-5' und 5'-TCAGTCG₁TTAG-X-GATTG₁CTGACT-5', worin G₁ = 2'-Desoxy-7-deazaguanosin, G₂ = Arabinoguanosin und X = Glycerinlinker, für eine Verwendung bei einer Erzeugung einer Immunreaktion in einem Vertebraten.

7. Oligonukleotid gemäß Anspruch 6, wobei der Verabreichungsweg ausgewählt ist aus parenteral, oral, sublingual, transdermal, topisch, intranasal, Aerosol, intraokular, intratracheal, intrarektal, vaginal, Genkanone, Hautpflaster, Augentropfen und Mundwasser.

8. Immunstimulierendes Oligonukleotid mit einer Struktur aus der Gruppe 5'-TCAGTCG₂TTAG-X-GATTG₂CTGACT-5' und 5'-TCAGTCG₁TTAG-X-GATTG₁CTGACT-5', worin G₁ = 2'-Desoxy-7-deazaguanosin, G₂ = Arabinoguanosin und X = Glycerinlinker, für eine Verwendung bei einer therapeutischen Behandlung eines Vertebraten mit Krebs, einer Autoimmunerkrankung, Atemwegsentzündung, entzündlichen Erkrankungen, Hauterkrankungen, Allergie, Asthma oder einer Erkrankung, die durch ein Pathogen verursacht wird.

9. Oligonukleotid gemäß Anspruch 8, wobei der Verabreichungsweg ausgewählt ist aus parenteral, oral, sublingual, transdermal, topisch, intranasal, Aerosol, intraokular, intratracheal, intrarektal, vaginal, Genkanone, Hautpflaster, Augentropfen und Mundwasser.

10. Immunstimulierendes Oligonukleotid mit einer Struktur aus der Gruppe 5'-TCAGTCG₂TTAG-X-GATTG₂CTGACT-5' und 5'-TCAGTCG₁TTAG-X-GATTG₁CTGACT-5', worin G₁ = 2'-Desoxy-7-deazaguanosin, G₂ = Arabinoguanosin und X = Glycerinlinker, für eine Verwendung bei einer Vermeidung von Krebs, einer Autoimmunerkrankung, Atemwegsentzündung, entzündlichen Erkrankungen, Hauterkrankungen, Allergie, Asthma oder einer Erkrankung, die durch ein Pathogen verursacht wird, bei einem Vertebraten.

11. Oligonukleotid gemäß Anspruch 10, wobei der Verabreichungsweg ausgewählt ist aus parenteral, oral, sublingual, transdermal, topisch, intranasal, Aerosol, intraokular, intratracheal, intrarektal, vaginal, Genkanone, Hautpflaster, Augentropfen und Mundwasser.

12. Oligonukleotid gemäß Anspruch 6, 8 oder 10, das zusammen mit einem Antikörper, Antisense-Oligonukleotid, Protein, Antigen, Allergen, Chemotherapeutikum oder Adjuvanz verabreicht werden soll.

13. Verwendung eines immunstimulierenden Oligonukleotids mit einer Struktur aus der Gruppe 5'-TCAGTCG₂TTAG-X-GATTG₂CTGACT-5' und 5'-TCAGTCG₁TTAG-X-GATTG₁CTGACT-5', worin G₁ = 2'-Desoxy-7-deazaguanosin, G₂ = Arabinoguanosin und X = Glycerinlinker, zur Herstellung einer pharmazeutischen Formulierung für eine Erzeugung einer Immunreaktion in einem Vertebraten.

14. Verwendung eines immunstimulierenden Oligonukleotids mit einer Struktur aus der Gruppe 5'-TCAGTCG₂TTAG-X-GATTG₂CTGACT-5' und 5'-TCAGTCG₁TTAG-X-GATTG₁CTGACT-5', worin G₁ = 2'-Desoxy-7-deazaguanosin, G₂ = Arabinoguanosin und X = Glycerinlinker, zur Herstellung einer pharmazeutischen Formulierung für eine therapeutische Behandlung eines Vertebraten mit Krebs, einer Autoimmunerkrankung, Atemwegsentzündung, entzündlichen Erkrankungen, Hauterkrankungen, Allergie, Asthma oder einer Erkrankung, die durch ein Pathogen verursacht wird.

15. Verwendung eines immunstimulierenden Oligonukleotids mit einer Struktur aus der Gruppe 5'-TCAGTCG₂TTAG-X-GATTG₂CTGACT-5' und 5'-TCAGTCG₁TTAG-X-GATTG₁CTGACT-5', worin G₁ = 2'-Desoxy-7-deazaguanosin, G₂ = Arabinoguanosin und X = Glycerinlinker, zur Herstellung einer pharmazeutischen Formulierung für eine Vermeidung von Krebs, einer Autoimmunerkrankung, Atemwegsentzündung, entzündlichen Erkrankungen, Hauterkrankungen, Allergie, Asthma oder einer Erkrankung, die durch ein Pathogen verursacht wird, bei einem Vertebraten.

16. Verwendung gemäß Anspruch 13, 14 oder 15, wobei das immunstimulierende Oligonukleotid zusammen mit einem Antikörper, Antisense-Oligonukleotid, Protein, Antigen, Allergen, Chemotherapeutikum oder Adjuvanz verabreicht werden soll.

## Revendications

1. Oligonucléotide immunostimulateur ayant une structure choisie dans le groupe de 5'-TCAGTCG₂TTAG-X-GATTG₂CTGACT-5' et 5'-TCAGTCG₁TTAG-X-GATTG₁CTGACT-5', où G₁ = 2'-désoxy-7-déazaguanosine; G₂ = arabinoguanosine; et X = lieur glycérol.

2. Composition comprenant l'oligonucléotide selon la revendication 1.

3. Composition selon la revendication 2, comprenant en outre un anticorps, oligonucléotide antisens, protéine, antigène, allergène, agent chimiothérapeutique ou adjuvant.

4. Formulation pharmaceutique comprenant l'oligonucléotide selon la revendication 1 et un support physiologiquement acceptable.

5. Formulation pharmaceutique selon la revendication 4, comprenant en outre un anticorps, oligonucléotide antisens, protéine, antigène, allergène, agent chimiothérapeutique ou adjuvant.

6. Oligonucléotide immunostimulateur ayant une structure choisie dans le groupe de 5'-TCAGTCG₂TTAG-X-GATTG₂CTGACT-5' et 5'-TCAGTCG₁TTAG-X-GATTG₁CTGACT-5', où G₁ = 2'-désoxy-7-déazaguanosine; G₂ = arabinoguanosine; et X = lieur glycérol, pour utilisation dans la production d'une réponse immune chez un vertébré.

7. Oligonucléotide selon la revendication 6, dans lequel la voie d'administration est choisie parmi les voies parentérale, orale, sublinguale, transdermique, topique, intranasale, en aérosol, intraoculaire, intratrachéale, intrarectale, vaginale, par pistolet à gènes, par timbre dermique, en gouttes oculaires et en bain de bouche.

8. Oligonucléotide immunostimulateur ayant une structure choisie dans le groupe de 5'-TCAGTCG₂TTAG-X-GATTG₂CTGACT-5' et 5'-TCAGTCG₁TTAG-X-GATTG₁CTGACT-5', où G₁ = 2'-désoxy-7-déazaguanosine; G₂ = arabinoguanosine; et X = lieur glycérol, pour utilisation dans le traitement thérapeutique d'un vertébré ayant un cancer, une affection auto-immune, une inflammation des voies aériennes, des troubles inflammatoires, des affections cutanées, une allergie, de l'asthme ou une affection provoquée par un agent pathogène.

9. Oligonucléotide selon la revendication 8, dans laquelle la voie d'administration est choisie parmi les voies parentérale, orale, sublinguale, transdermique, topique, intranasale, en aérosol, intraoculaire, intratrachéale, intrarectale, vaginale, par pistolet à gènes, par timbre dermique, en gouttes oculaires et en bain de bouche.

10. Oligonucléotide immunostimulateur ayant une structure choisie dans le groupe de 5'-TCAGTCG₂TTAG-X-GATTG₂CTGACT-5' et 5'-TCAGTCG₁TTAG-X-GATTG₁CTGACT-5', où G₁ = 2'-désoxy-7-déazaguanosine; G₂ = arabinoguanosine; et X = lieur glycérol, pour utilisation dans la prévention d'un cancer, d'une affection auto-immune, d'une inflammation des voies aériennes, de troubles inflammatoires, d"affections cutanées, d'une allergie, de l'asthme ou d'une affection provoquée par un agent pathogène chez un vertébré.

11. Oligonucléotide selon la revendication 10, dans laquelle la voie d'administration est choisie parmi les voies parentérale, orale, sublinguale, transdermique, topique, intranasale, en aérosol, intraoculaire, intratrachéale, intrarectale, vaginale, par pistolet à gènes, par timbre dermique, en gouttes oculaires et en bain de bouche.

12. Oligonucléotide selon la revendication 6, 8 ou 10, qui est destiné à être administré conjointement avec un anticorps, oligonuélcotide antisens, protéine, antigène, allergène, agent chimiothérapeutique ou adjuvant.

13. Utilisation d'un oligonucléotide immunostimulateur ayant une structure choisie dans le groupe de 5'-TCAGTCG₂TTAG-X-GATTG₂CTGACT-5' et 5'-TCAGTCG₁TTAG-X-GATTG₁CTGACT-5', où G₁ = 2'-désoxy-7-déazaguanosine; G₂ = arabinoguanosine; et X = lieur glycérol, pour la préparation d'une formulation pharmaceutique pour la production d'une réponse immune chez un vertébré.

14. Utilisation d'un oligonucléotide immunostimulateur ayant une structure choisie dans le groupe de 5'-TCAGTCG₂TTAG-X-GATTG₂CTGACT-5' et 5'-TCAGTCG₁TTAG-X-GATTG₁CTGACT-5', où G₁ = 2'-désoxy-7-déazaguanosine; G₂ = arabinoguanosine; et X = lieur glycérol, pour la préparation d'une formulation pharmaceutique pour le traitement thérapeutique d'un vertébré ayant un cancer, une affection auto-immune, une inflammation des voies aériennes, des troubles inflammatoires, des affections cutanées, une allergie, de l'asthme ou une affection provoquée par un agent pathogène.

15. Utilisation d'un oligonucléotide immunostimulateur ayant une structure choisie dans le groupe de 5'-TCAGTCG₂TTAG-X-GATTG₂CTGACT-5' et 5'-TCAGTCG₁TTAG-X-GATTG₁CTGACT-5', où G₁ = 2'-désoxy-7-déazaguanosine; G₂ = arabinoguanosine; et X = lieur glycérol, pour la préparation d'une formulation pharmaceutique pour la prévention du cancer, d'une affection auto-immune, d'une inflammation des voies aériennes, des troubles inflammatoires, d'affections cutanées, d'une allergie, de l'asthme ou d'une affection provoquée par un agent pathogène chez un vertébré.

16. Utilisation selon la revendication 13, 14 ou 15, dans laquelle l'oligonucléotide immunostimulateur est destiné à être administré avec un anticorps, oligonucléotide antisens, protéine, antigène, allergène, agent chimiothérapeutique ou adjuvant.
